# EUROPÄISCHE PATENTSCHRIFT

(11) **EP 2 412 400 B1**
(45) Veröffentlichungstag und Bekanntmachung des Hinweises auf die Patenterteilung: **21.08.2013**
(21) Anmeldenummer: 11172361.5
(22) Anmeldetag: 01.07.2011
(51) Int. Cl.: A61N 1/05, H01R 4/50, A61N 1/02

(54) **Elektrodenanbindung, insbesondere für einen Elektrodenkatheter**
Electrode connection, in particular for an electrode catheter
Raccordement électrique, notamment pour un cathéter à électrodes

(30) Priorität: 27.07.2010 US 367889 P
(43) Veröffentlichungstag der Anmeldung: 01.02.2012
(73) Patentinhaber: BIOTRONIK SE & Co. KG, 12359 Berlin (DE)
(72) Erfinder: Weitzig, Pierre, 10249 Berlin (DE); Palm, Jochen, 15831 Mahlow (DE); Jadwizak, Dietmar, 15537 Erkner (DE); Fründt, Carsten, 13057 Berlin (DE)
(74) Vertreter: Lindner-Vogt, Karin L.

(56) Entgegenhaltungen:
- DE-U1-202005 020 835
- US-A- 4 566 467
- US-A- 5 569 883
- US-A1- 2005 228 469
- US-B1- 6 505 081

## Beschreibung

Die Erfindung betrifft eine formschlüssige Elektrodenanbindung, insbesondere für einen Elektrodenkatheter, die eine Elektrode, eine als Wendel ausgebildete, mit der Elektrode verbundene Leitung für elektrische Signale und eine Fixiereinrichtung für das elektrodenseitige Ende der Wendel an der Elektrode, welche eine Innenhülse, auf der das elektrodenseitige Ende der Wendel sitzt, und einen Quetschring, der das elektrodenseitige Ende der Wendel unter Herstellung eines elektrischen Kontaktes mit der Elektrode und mechanischer Klemmung auf der Innenhülse beaufschlagt, aufweist, umfasst.

Zum Hintergrund der Erfindung ist festzuhalten, dass die mechanische und elektrische Verbindung der als Wendel ausgelegten Leitung in einem Elektrodenkatheter mit einer Kopf- oder Ringelektrode - beispielsweise in einer Herzschrittmacherelektrode - besonders zuverlässig sein muss, da eine Unterbrechung dieser Verbindung zu einem Ausfall des Elektrodenkatheters führt, was für den Patienten fatale Folgen haben kann. Ferner bedeutet ein solcher Defekt die Notwendigkeit, den Elektrodenkatheter auszuwechseln, was einen Eingriff mit allen üblichen Begleitumständen und Folgen für den Patienten notwendig macht.

Durch offenkundige Vorbenutzungen sind grundsätzlich mechanische und elektrische Verbindungen der Wendel mit der Elektrode auf der Basis von Schweiß- und Crimpprozessen bekannt und üblich. Wenngleich derartige Verbindungen hinsichtlich ihrer Zuverlässigkeit durchaus akzeptabel sind, weisen diese bekannten Lösungen doch verschiedene Nachteile auf.

Beim Schweißen muss der Wendelwerkstoff naturgemäß grundsätzlich schweißbar sein. Insoweit sind Schweißprozesse nicht anwendbar bei Wendeldrähten aus verschiedenen Kern- und Mantelmaterialien, welche über unterschiedliche Schmelztemperaturen verfügen. Ferner müssen auch die zu verbindenden Werkstoffe so aufeinander abgestimmt sein, dass sie miteinander verschweißbar sind. Dies ist abhängig von den Schmelztemperaturen der Schweißpartner und der Diffusion von Legierungselementen darin. Dies kann zur Versprödung der Fügepartner im Bereich der Wärmeeinflusszone führen. Sehr dünnwandige Bauteile innerhalb der Elektrodenvorrichtung lassen sich grundsätzlich nur sehr problematisch verschweißen. Ein weiterer Nachteil ist die schlechte Überprüfmöglichkeit der Schweißverbindung. Eine Erstprüfung auf mechanische Festigkeit und elektrischen Kontakt kann auch bei einer auf Dauer mangelhaften Verschweißung durchaus positiv ausfallen. Schließlich können geringste Verunreinigungen an den Bauteilen zu unvollständigen Schweißverbindungen führen und demzufolge Fertigungsausschuss zur Folge haben.

Das oben erwähnte Crimpen weist dahingehend produktionstechnische Sicherheitsdefizite auf, als der Grad der Verbindungsqualität in der Regel abhängig von der Härte der Bauteile ist. Ferner sind kleine Durchmesser an den bei der Crimpverbindung beteiligten Komponenten nicht realisierbar, da bestimmte Mindestwandstärken und eine an die Crimptechnik adaptierte Konstruktion der Verbindung mit einem notwendigen Unterbau und einer gesonderten Crimphülse gegeben sein müssen.

Die Dokumente US 2005/0228469, DE 20 2005 020835 U1, US 6,505,081, US 4,566,467 und US 5,569,883 offenbaren allesamt den eingangs genannten Oberbegriff des Anspruchs 1.

Ausgehend von den geschilderten Problemen des Standes der Technik liegt der Erfindung die Aufgabe zugrunde, eine Elektrodenanbindung in ihrer Fixiereinrichtung für das elektrodenseitige Ende der Wendel an der Elektrode so zu verbessern, dass eine sichere mechanische und elektrische Verbindung der Wendel mit der Elektrode in werkstoffunabhängiger Weise hergestellt werden kann.

Diese Aufgabe wird durch eine Elektrodenanbindung mit einer Fixiereinrichtung erfüllt, bei der die Innenhülse einen sich von ihrem in die Wendel eingreifenden Ende ausgehend erweiternden Außenkonus aufweist, auf dem das Wendelende ebenfalls konisch aufgeweitet sitzt, wobei der Quetschring mit einem axial auf das aufgeweitete Wendelende geschobenen Innenkonus das Wendelende festlegt, und wobei er an seiner radial nach außen weisenden Seite mit der Elektrode mechanisch und elektrisch leitend verbunden ist.

Die erfindungsgemäße Elektrodenanbindung vermeidet erkennbar die Probleme des geschilderten Standes der Technik. So wird zum einen keine Schweißverbindung eingesetzt, wodurch die Fixiereinrichtung eine werkstoffunabhängige Verbindung zwischen der Drahtwendel und der Elektrode herstellen kann. Da es ein rein mechanischer Prozess ist, ist zum anderen eine bessere Überwachbarkeit gegeben, was grundsätzlich zu einer Qualitätsverbesserung der Verbindung führt.

Die Ansprüche 2 bis 5 kennzeichnen eine erste vorteilhafte Umsetzung des Grundprinzips der Elektrodenanbindung, wie es im Anspruch 1 angegeben ist. Diese Variante beruht auf einer Art Formschluss zwischen dem durch den Außenkonus der Innenhülse aufgeweiteten Wendelende mit dem mit einem entsprechenden Innenkonus versehenen Quetschring, der auf das aufgeweitete Wendelende aufgeschoben ist. Insbesondere wird der Quetschring unter Deformation der das Wendelende bildenden Leitung aufgeschoben, sodass eine innige, feste Verbindung nach Art eines Formschlusses zwischen Innenhülse, Drahtwendel und Quetschring hergestellt wird.

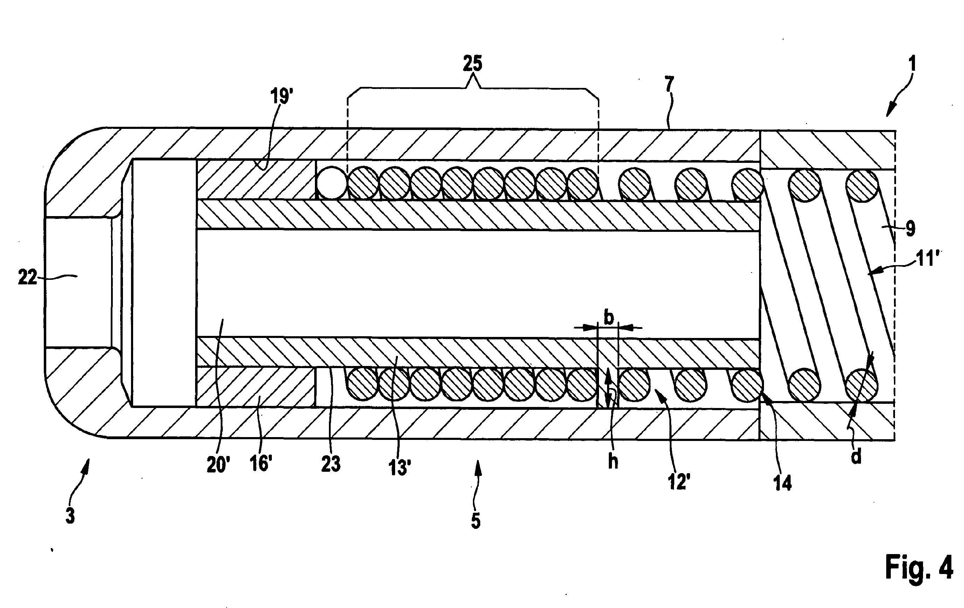

Eine zum technischen Hintergrund der Patentanmeldung zählende Variante des Grundkonzepts, wie sie in den Fig. 4 bis 6 dargestellt ist, beruht auf einer Art Schraubverbindung, indem das elektrodenseitige Ende der offen gewickelten Wendel auf die Innenhülse durch Hintergreifen eines Gewindeelements an dieser aufgeschraubt und die über das Gewindeelement geschraubte Partie des Wendelelements durch den Quetschring insbesondere axial gegen das Gewindeelement beaufschlagt ist. Durch die damit hervorgerufene Stauchung des offen gewickelten Wendelendes insbesondere bis auf Block durch den Quetschring wird eine Art Selbsthemmung in der Schraubverbindung zwischen der Drahtwendel und der Innenhülse hervorgerufen, die ein Lösen der mechanischen und damit elektrischen Verbindung zwischen Innenhülse und Wendelende grundsätzlich unmöglich macht.

Weitere Merkmale, Einzelheiten und Vorteile der Erfindung ergeben sich aus der nachfolgenden Beschreibung von Ausführungsbeispielen anhand der beigefügten Zeichnungen. Es zeigen:
- Fig. 1: eine Seitenansicht eines Elektrodenkatheters,
- Fig. 2 und 3: vergrößerte, ausschnittsweise Längsaxialschnitte des Elektrodenkatheters im Bereich einer Ring- und Kopfelektrode in einer ersten Ausführungsform,
- Fig.4: einen vergrößerten, ausschnittsweisen Längsaxialschnitt eines Elektrodenkatheters im Bereich der Kopfelektrode in einer zum technischen Hintergrund der Patentanmeldung gehörenden Ausführungsform,
- Fig. 5: eine Seitenansicht der in der Fixiereinrichtung gemäß Fig. 4 verwendeten Innenhülse, und
- Fig. 6: eine Axialansicht der Innenhülse gemäß Fig. 5.

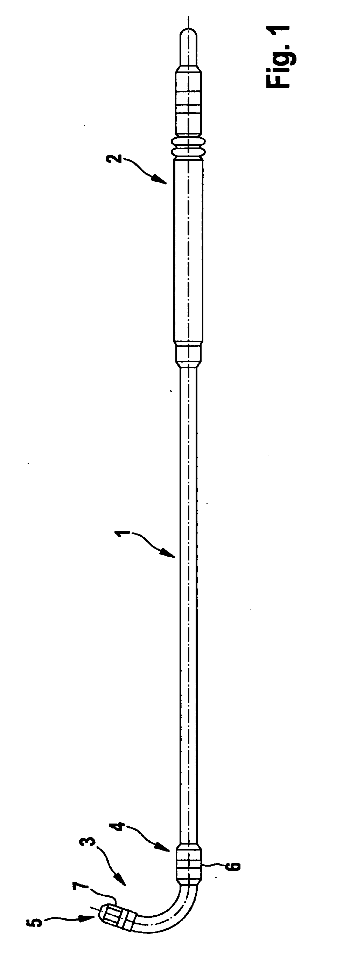

Wie insbesondere aus Figur 1 hervorgeht, weist der zur Kardialtherapie dienende Elektrodenkatheter einen langgestreckten, schlauchartigen Katheterkörper 1 auf, der an seinem proximalen Ende mit einem Anschlussstecker 2 zur Verbindung mit einem entsprechenden Implantat versehen ist. Im Bereich des distalen Endes 3 ist eine Ringelektrode 4 sowie unmittelbar an der Spitze des distalen Endes 3 eine Kopfelektrode 5 angeordnet. Diese Elektroden 4, 5 dienen zur Abgabe oder Messung eines elektrischen Signals, im vorliegenden Falle also eines elektrokardialen Signals über ihre äußere Elektrodenkontaktfläche 6, 7 beispielsweise für eine zuverlässige und effektive Defibrillation oder eine Diagnostik zur Früherkennung von Vorhofflimmern und einer Herzinsuffizienzprogression.

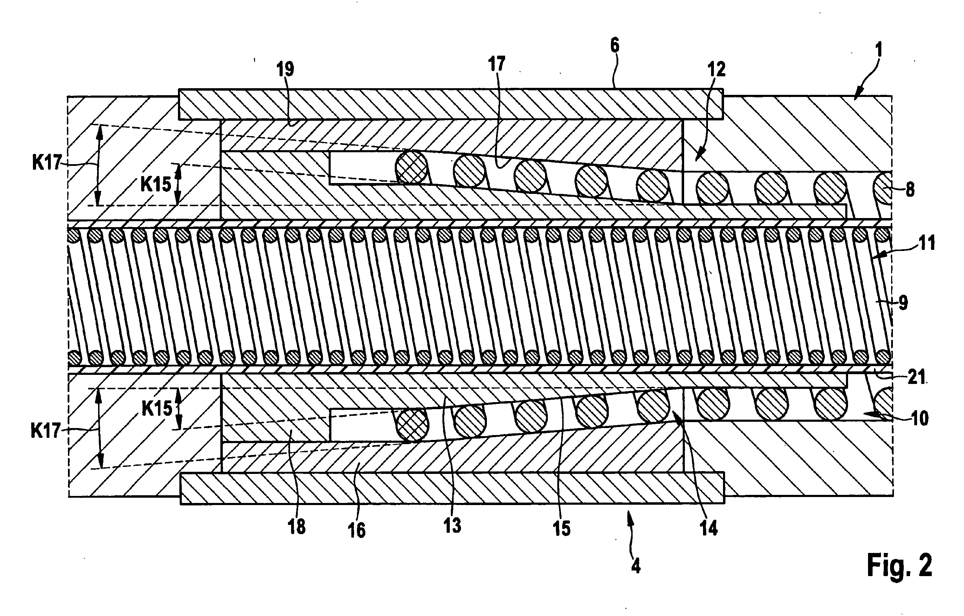

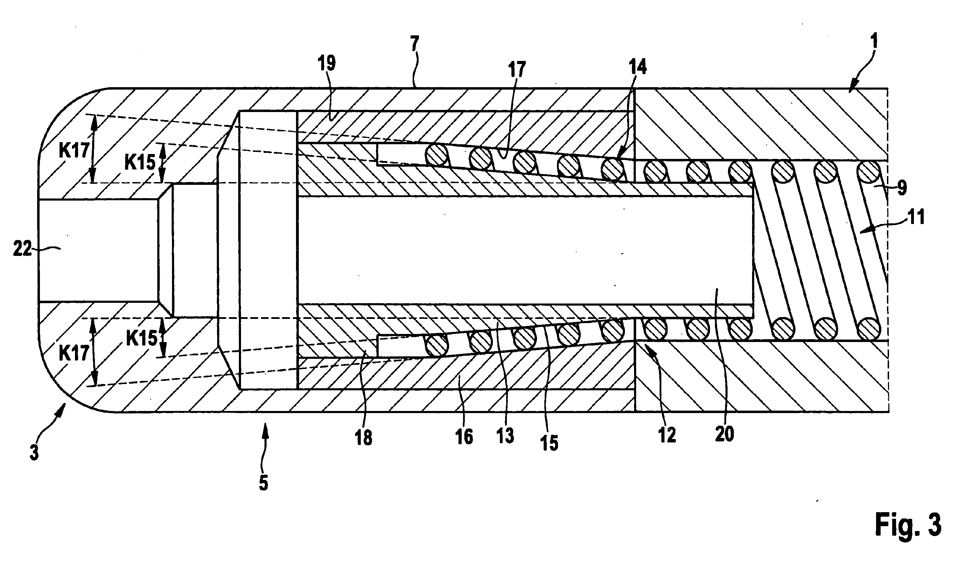

Anhand von Fig. 2 und 3 ist nur eine erste Ausführungsform für die Anbindung der Elektroden 4, 5 an entsprechende Zuleitungen 8, 9 zu erläutern. So werden in dem schlauchartigen Katheterkörper 1 von proximal her zwei solche Zuleitungen 8, 9 in Form von koaxial ineinander sitzenden Wendeln 10, 11 herangeführt. Die äußere Wendel 10 mit der Zuleitung 8 dient zur Verbindung mit der Ringelektrode 4, wie sie in Fig. 2 dargestellt ist. Die innere Wendel 11 wird in distale Richtung durch die Ringelektrode 4 isoliert weitergeführt.

Die in Fig. 2 dargestellte, als Ganzes mit 12 bezeichnete, Fixiereinrichtung weist eine Innenhülse 13 auf, auf der das elektrodenseitige Ende 14 der Wendel 10 sitzt. Die Innenhülse 13 ist dabei mit einem sich in distaler Richtung erweiternden Außenkonus 15 versehen, auf den bei der Montage das Wendelende 14 aufgeschoben wird und damit ebenfalls konisch aufgeweitet sitzt.

Auf die Baugruppe aus Innenhülse 13 und elektrodenseitigem Ende 14 der Wendel 10 ist ein Quetschring 16 aufgeschoben, der mit einem sich ebenfalls in distaler Richtung aufweitenden Innenkonus 17 versehen ist. Der Quetschring 16 wird vor dem Aufschieben des elektrodenseitigen Endes 14 der Wendel 10 auf diese aufgefädelt und nach dem Verbinden der Wendel 10 mit der Innenhülse 13 in distaler Richtung nachgeschoben und auf das aufgeweitete Wendelende quasi aufgequetscht. Dabei wird die das Wendelende 14 bildende Zuleitung 8 etwas deformiert, sodass eine Art Formschluss zwischen Innenhülse 13, Wendelende 14 und Quetschring 16 unter Erzielung einer mechanisch stabilen und elektrisch sicheren Verbindung zwischen diesen aus elektrisch leitfähigem Werkstoff bestehenden Bauteilen hergestellt wird. Im distalen Endbereich wird der Quetschring 16 auf einer Lagerschulter 18 am distal weisenden Ende der Innenhülse 13 durch Aufsetzen fixiert.

Die Konuswinkel K15 bzw. K17 am Außenkonus 15 der Innenhülse 13 bzw. am Innenkonus 17 des Quetschrings 16 sind flach und liegen insbesondere unter einem Betrag von 15°, vorzugsweise beispielsweise bei 10°. Ferner stimmen sie in ihrem Winkelbetrag überein.

Zur Herstellung einer elektrischen Verbindung zwischen der Wendel 10 und der Ringelektrode 4 ist der Quetschring 16 an seiner radial nach außen weisenden Seite 19 mit der Ringelektrode 4 elektrisch leitend, beispielsweise eine stoffschlüssige Verbindung, verbunden.

Durch die Innenbohrung 20 der Innenhülse 13 ist unter Zwischenlage eines isolierenden Schlauchs 21 die innere Wendel 11 der Zuleitung 9 in distale Richtung zur Kopfelektrode 5 (Fig. 3) weitergeführt.

In Fig. 3 ist eine Fixiereinrichtung 12 analog der anhand von Fig. 2 erläuterten Fixiereinrichtung zur Anbindung der inneren Wendel 11 mit der Zuleitung 9 an die Kopfelektrode 5 gezeigt. Der Aufbau stimmt mit der Fixiereinrichtung 12 gemäß Fig. 2 überein, insoweit ist die Beschreibung der Fixiereinrichtung 12 von Fig. 2 in vollem Umfang auf die in Fig. 3 gezeigte Konstruktion übertragbar, zur Vermeidung von Wiederholungen kann darauf verwiesen werden. Konstruktiv übereinstimmende Bauteile sind mit identischen Bezugszeichen versehen.

Die Innenbohrung 20 der Innenhülse 13 bei der Fixiereinrichtung 12 an der Kopfelektrode 5 dient zum Durchführen eines Mandrins bzw. von Führungsdrähten für den Elektrodenkatheter. Mit der Innenbohrung 20 fluchtet dabei eine zentrale Kopfbohrung 22 in der Kopfelektrode 5.

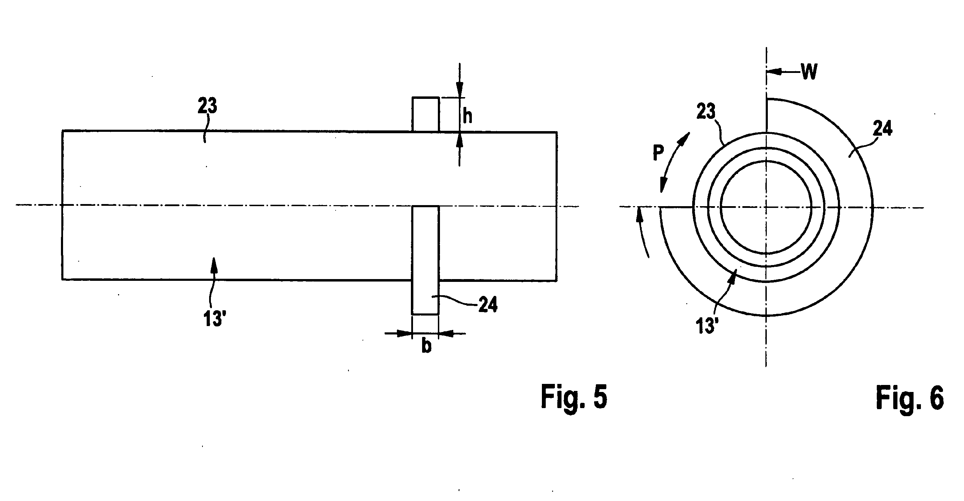

In den Fig. 4 bis 6 ist eine zum technischen Hintergrund der Patentanmeldung zählende Ausführungsform einer Fixiereinrichtung 12' für die Verbindung einer Wendel 11 der Zuleitung 9 mit der Kopfelektrode 5 gezeigt. Diese weist eine Innenhülse 13' auf, die nicht als Konus, sondern hinsichtlich ihres Grundkörpers als Zylinderhülse ausgeführt ist. Bei etwa einem Viertel ihrer Gesamtlänge ist auf der Mantelfläche 23 der Innenhülse 13' ein Gewindeelement in Form eines über einen Teilumfang der Innenhülse 13' verlaufenden Stegs 24 ausgebildet. Wie insbesondere aus Fig. 5 und 6 deutlich wird, verläuft dieser Steg 24 über einen Winkel W von 270° in Peripherrichtung P um diese Innenhülse 13'. Der Steg weist eine Höhe h auf, die etwas größer als der Durchmesser d der die Wendel bildenden Zuleitung 9 ist. Seine Breite b entspricht etwa diesem Durchmesser d.

Wie aus Fig. 4 deutlich wird, ist die Wendel 11' mit Windungslücken versehen und kann somit auf die Innenhülse 13' über den Gewindeelement-Steg 24 aufgeschraubt werden, bis mehrere Windungen den Steg 24 passiert haben. Dann wird auf das distale Ende der Innenhülse 13' ein Quetschring 16' gegen diese aufgeschraubten Wickelwindungen 25 aufgeschoben, sodass diese Wickelwindungen 25 so beaufschlagt werden, dass sie auf Block gestaucht sind. Dieser Zustand ist in Fig. 4 dargestellt. Aufgrund dieser Stauchung ist das Windungspaket 25 zwischen Quetschring 16' und Steg 24 so durch Selbsthemmung fixiert, dass ein Herausschrauben der Wendel 11' und damit eine Lösung der Verbindung zwischen dieser und der Innenhülse 13' unmöglich ist.

Der Quetschring 16' wird durch geeignete Maßnahmen, wie beispielsweise eine stoffschlüssige Verbindung, auf der Innenhülse 13' fixiert. Gleichermaßen ist wiederum die radial nach außen weisende Seite 19' des Quetschrings 16' mit der Kopfelektrode 5 durch eine stoffschlüssige Verbindung elektrisch und mechanisch sicher verbunden.

Die Innenbohrung 20' der Innenhülse 13' dient zusammen mit der Kopfbohrung 22 der Kopfelektrode 5 wiederum zum Durchführen eines Mandrins oder von Führungsdrähten für den Elektrodenkatheter.

## Patentansprüche

1. Elektrodenanbindung, insbesondere für einen Elektrodenkatheter, umfassend
- eine Elektrode (4, 5),
- eine als Wendel (10, 11, 11') ausgebildete, mit der Elektrode (4, 5) verbundene Leitung (8, 9) für elektrische Signale, und
- eine Fixiereinrichtung (12, 12') für das elektrodenseitige Ende (14) der Wendel (10, 11, 11') an der Elektrode, welche aufweist;
= eine Innenhülse (13, 13'), auf der das elektrodenseitige Ende (14) der Wendel (10, 11, 11') sitzt, und
= einen Quetschring (16, 16'), der das elektrodenseitige Ende (14) der Wendel (10, 11, 11') unter Herstellung eines elektrischen Kontaktes mit der Elektrode (4, 5) und mechanischer Klemmung auf der Innenhülse (13, 13') beaufschlagt;
**dadurch gekennzeichnet, dass** die Innenhülse (13) einen sich von ihrem in die Wendel (10, 11) eingreifenden Ende ausgehend erweiternden Außenkonus (15) aufweist, auf dem das Wendelende (14) ebenfalls konisch aufgeweitet sitzt, wobei der Quetschring (16) mit einem axial auf das aufgeweitete Wendelende (14) geschobenen Innenkonus (17) das Wendelende (14) festlegt, und wobei er an seiner radial nach außen weisenden Seite (19, 19') mit der Elektrode (4, 5) mechanisch und elektrisch leitend verbunden ist.

2. Elektrodenanbindung nach Anspruch 1, **dadurch gekennzeichnet, dass** der Quetschring (16) unter Deformation der das Wendelende (14) bildenden Leitung (8) aufgeschoben ist.

3. Elektrodenanbindung nach Anspruch 1 oder 2, **dadurch gekennzeichnet, dass** der Quetschring (16) in seiner Fixierstellung auf einer Lagerschulter (18) an der Innenhülse (13) befestigt ist.

4. Elektrodenanbindung nach einem der Ansprüche 1 bis 3, **dadurch gekennzeichnet, dass** die Konuswinkel (K15 bzw. K17) am Außenkonus (15) der Innenhülse (13) und am Innenkonus (17) des Quetschrings (16) kleiner als 15° sind.

5. Elektrodenanbindung nach Anspruch 4, **dadurch gekennzeichnet, dass** die Konuswinkel (K15, K17) am Außenkonus (15) der Innenhülse (13) und am Innenkonus (17) des Quetschrings (16) übereinstimmen.

## Claims

1. An electrode connection, in particular for an electrode catheter, comprising
- an electrode (4, 5),
- a line (8, 9) for electrical signals, said line being designed as a coil (10, 11, 11') and being connected to the electrode (4, 5), and
- a fixation device (12, 12') for the electrode-side end (14) of the coil (10, 11, 11') on the electrode, said fixation device comprising;
= an inner sleeve (13, 13'), on which the electrode-side end (14) of the coil (10, 11, 11') sits, and
= a squeeze ring (16, 16'), which acts upon the electrode-side end (14) of the coil (10, 11, 11'), establishing an electrical contact with the electrode (4, 5) and mechanical clamping on the inner sleeve (13, 13');
**characterised in that** the inner sleeve (13) has an outer cone (15), which widens starting from its end engaging into the coil (10, 11) and on which the coil end (14) also sits in a conical widened manner, wherein the squeeze ring (16) secures the coil end (14) to an inner cone (17), which is slid axially onto the widened coil end (14), and wherein said squeeze ring is mechanically and electrically conductively connected on its radially outwardly pointing side (19, 19') to the electrode (4, 5).

2. The electrode connection according to Claim 1, **characterised in that** the squeeze ring (16) is slid on with deformation of the line (8) forming the coil end (14).

3. The electrode connection according to Claim 1 or 2, **characterised in that** the squeeze ring (16) is fastened in its fixing position on a bearing shoulder (18) to the inner sleeve (13).

4. The electrode connection according to one of Claims 1 to 3, **characterised in that** the cone angles (K15 and K17) on the outer cone (15) of the inner sleeve (13) and on the inner cone (17) of the squeeze ring (16) are smaller than 15°.

5. The electrode connection according to Claim 4, **characterised in that** the cone angles (K15, K17) on the outer cone (15) of the inner sleeve (13) and on the inner cone (17) of the squeeze ring (16) are equal.

## Revendications

1. Raccordement d'électrodes, en particulier pour un cathéter à électrodes, comprenant
- une électrode (4, 5),
- une ligne (8, 9) pour signaux électriques, conçue comme un filament (10, 11, 11') et reliée à l'électrode (4, 5), et
- un dispositif de fixation (12, 12') pour l'extrémité côté électrode (14) du filament (10, 11, 11') sur l'électrode, comprenant :
= une douille intérieure (13, 13') sur laquelle est appliquée l'extrémité côté électrode (14) du filament (10, 11, 11'), et
= une bague de serrage (16, 16') sollicitant l'extrémité côté électrode (14) du filament (10, 11, 11') en établissant un contact électrique avec l'électrode (4, 5) et un serrage mécanique sur la douille intérieure (13, 13') ;
**caractérisé en ce que** la douille intérieure (13) comporte un cône extérieur (15) s'élargissant à partir de son extrémité engagée dans le filament (10, 11), sur lequel l'extrémité de filament (14) s'applique en s'élargissant également de façon conique, la bague de serrage (16) fixant l'extrémité de filament (14) avec un cône intérieur (17) enfoncé axialement sur l'extrémité de filament (14) élargie, tout en étant reliée de façon mécanique et électriquement conductrice à l'électrode (4, 5), par son côté (19, 19') tourné radialement vers l'extérieur.

2. Raccordement d'électrodes selon la revendication 1, **caractérisé en ce que** la bague de serrage (16) est enfoncée en déformant la ligne (8) formant l'extrémité de filament (14).

3. Raccordement d'électrodes selon la revendication 1 ou 2, **caractérisé en ce que** dans sa position de fixation, la bague de serrage (16) est fixée à la douille intérieure (13) par une épaule de support (18).

4. Raccordement d'électrodes selon l'une des revendications 1 à 3, **caractérisé en ce que** les angles de cône (K15 ou K17) sur le cône extérieur (15) de la douille intérieure (13) et sur le cône intérieur (17) de la bague de serrage (16) sont inférieurs à 15°.

5. Raccordement d'électrodes selon la revendication 4, **caractérisé en ce que** les angles de cône (K15, K17) sur le cône extérieur (15) de la douille intérieure (13) et sur le cône intérieur (17) de la bague de serrage (16) coïncident l'un avec l'autre.
